# EUROPÄISCHE PATENTSCHRIFT

(11) **EP 0 325 194 B1**
(45) Veröffentlichungstag und Bekanntmachung des Hinweises auf die Patenterteilung: **03.11.1993**
(21) Anmeldenummer: 89100683.5
(22) Anmeldetag: 17.01.1989
(51) Int. Cl.: F16K 17/06

(54) **Schaltbares Ueberdruckventil für strömende Medien**
Change-over relief valve for flowing fluids
Soupape de sûreté renversable pour fluides coulants

(30) Priorität: 20.01.1988 DE 3801444
(43) Veröffentlichungstag der Anmeldung: 26.07.1989
(73) Patentinhaber: Drägerwerk Aktiengesellschaft, 23542 Lübeck (DE)
(72) Erfinder: Kleinschmidt, Lothar, Dipl.-Ing., D-2401 Krummesse (DE); Waschmann, Michael, Dr. Ing., D-2400 Lübeck (DE)

(56) Entgegenhaltungen:
- DE-A- 3 006 409
- DE-B- 1 181 516
- DE-B- 2 649 033
- DE-U- 1 998 797
- FR-A- 2 390 651
- FR-A- 2 503 319
- GB-A- 1 060 471
- US-A- 3 780 760

## Beschreibung

Die Erfindung betrifft ein Überdruckventil entsprechend den im Oberbegriff von Anspruch 1 aufgefürten Merkmalen, die aus der DE-B 1181516 bekannt sind.

Ein weiteres Überdruckventil ist aus der US-A 3 780 760 bekanntgeworden. Dieses bekannte Ventil wird vorzugsweise in einer Astemgasleitung für Anästhesie- oder Beatmungsgeräte eingesetzt, um u. a. zwischen den zwei Betriebszuständen "maschinelle Beatmung" und "spontane Atmung" bzw. "Handbeatmung" eine entsprechende Schaltstellung zu ermöglichen. So ist das Ventil in der Betriebsart "maschinelle Beatmung" geschlossen und wird entgegen einer voreingestellten Schließkraft durch einen möglichen Überdruck in der Atemgasleitung geöffnet. Dadurch wird der Überdruck in der Atemgasleitung abgebaut bzw. überschüssiges Atemgas kann in die Umgebung abgegeben werden. In der Betriebsart "Spontanatmung" bzw. "Handbeatmung" muß die Verbindung der Atemgasleitung während der Exspiration von dem angeschlossenen Beatmungs- bzw. Narkosebeatmungsgerät abgetrennt werden, so daß das eingeatmete Gasgemisch an die Umgebung abgegeben werden kann. Dazu ist bei dem bekannten Ventil eine Verstelleinrichtung vorgesehen, durch deren Betätigung das Ventil in eine geöffnete Stellung anlüftbar ist. Dadurch wird der Ausatemwiderstand so gering, daß eine spontane Ausatmung erfolgen kann. Während einer Narkosebehandlung und auch im Zuge einer länger andauernden maschinellen Beatmung ist eine wiederholte Umschaltung des Ventils von seinem geschlossenen in seinen angelüfteten Zustand und umgekehrt notwendig, insbesondere dann, wenn de an das betreffende Gerät angeschlossene Patient von der künstlichen Beatmung entwöhnt und seine natürliche Atemtätigkeit wieder wachgerufen werden soll.

Bei diesem bekannten Ventil wird während der Verstellung vom geschlossenen in den angelüfteten Zustand und umgekehrt gleichzeitig die über eine Feder eingestellte Schließkraft für das Verschlußelement verstellt, so daß beim nachträglichen Wiederverschließen die vorher eingestellte Schließkraft ebenfalls neu eingestellt werden muß. Dabei kann es passieren, daß der Bediener den ursprünglichen Einstellwert für die Schließkraft vergessen hat und nunmehr eine langwierige Abstimmung von Atemwegsdruck während der maschinellen Beatmung und entsprechend einzustellendem Überdruck durchführen muß. Dies ist aber bei der in den angesprochenen Betriebsarten erforderlichen Aufmerksamkeit für den übrigen Arbeitsablauf nicht voraussetzbar. Man erwartet vielmehr ein mit einfachen und sicheren Handgriffen betätigbares Verstellen eines solchen Ventils, dem der Bediener keine besondere Aufmerksamkeit widmen muß.

Der vorliegenden Erfindung liegt somit die Aufgabe zugrunde, ein Überdruckventil der genannten Art so zu verbessern, daß die Umstellung von der geschlossenen in die angelüftete Position und umgekehrt ohne Beeinflussung der Einstellung für die Schließkraft erfolgen kann, und daß die Verstellung der Schließkraft in beiden Positionen möglich sein soll. Die Umschaltung soll zudem sicher, einfach und rasch handhabbar sein.

Die Lösung der Aufgabe erfolgt mit den im Anspruch 1 aufgeführten Merkmalen.

Die mit der Erfindung erzielten Vorteile liegen im wesentlichen darin, daß eine Umschaltung aus dem geschlossenen in den angelüfteten Zustand des Ventils ohne Beeinflussung der einmal eingestellten Schließkraft erfolgen kann. Bei der Rückkehr der Schaltvorrichtung in ihre Schließstellung kann die einmal eingestellte Schließkraft auf das Verschlußelement unverändert einwirken, und es wird wieder mit demselben Anpreßdruck gegen seinen Sitz gedrückt, wie es vor Überführung in die Lüftstellung der Fall gewesen war. Der Wechsel der Schaltstellung kann mit einem einzigen Handgriff erfolgen, der je nach Ausgestaltung der dafür notwendigen Betätigungselemente sogar blind durchgeführt werden kann. Das Schalten ist außerdem hörbar und gibt dem Betreiber somit ein akustisches Zeichen der erfolgten Umstellung.

Die Umschaltung in die angelüftete Position erfolgt nicht mehr durch gleichzeitiges Verstellen der Schließkraft auf das Verschlußelement. Will man hingegen die Schließkraft verstellen, so kann dieses in beiden Positionen des Verschlußelementes erfolgen.

Da die Verstelleinrichtung innerhalb einer drehbaren Ventilspindel und mit dieser über die Schaltvorrichtung verbunden einen mittels eines Betätigungsstückes hubbeweglichen Ventilschaft enthält, dient dieser Ventilschaft der Verbindung zwischen dem Verschlußelement, z.B. einem Ventilteller, und dem Betätigungsstück für die Verstelleinrichtung. Für die Schließkraft des Ventiltellers auf seinen Sitz sorgt ein Schließfederelement, welches zwischen dem Ventilteller und einem den Schaft umgreifenden Haltekragen angreift. Die Ankopplung des Ventiltellers über eine Schleppverbindung erlaubt diesem in der Schließstellung eine freie, nur durch die gewählte Einstellung der Schließfeder bestimmte Beweglichkeit. Zwischen der Ventilspindel und dem Ventilschaft greift ein weiteres Federelement an, dessen Kraftwirkung einen der beiden Schaltzustände hervorbringt und im anderen durch die Schaltvorrichtung überwunden wird.

In weiterer Ausgestaltung der Erfindung kann vorgesehen sein, die Ventilspindel mit einem Drehstück als Handhabe auszustatten, z.B. in Form eines Handrades. Damit ergibt sich eine sinnfällige Zuordnung des Betätigungsorgans zur Einstellfunktion für die Schließkraft. Auf der mit einem Außengewinde versehenen Ventilspindel ist der mit einem entsprechenden Innengewinde versehene Haltekragen angeordnet . Um den Haltekragen bezüglich einer Drehung der Ventilspindel festzuhalten und gleichzeitig in Längsrichtung entlang der Ventilspindel verschiebbar auszuführen, ist der Haltekragen mit Fahnen versehen, welche in Führungsnuten greifen.

In weiterer Ausbildung der Erfindung weist die Schaltvorrichtung ein von der Ventilspindel getragenes Rastgehäuse und in dieses eingreifende, mit dem Ventilschaft verbundene Rastnocken auf. Die Verklammerung von Ventilspindel und Ventilschaft über Rastnocken bildet eine sehr einfach zu handhabende druckbetätigte Rastvorrichtung. Das Betätigungsstück ist als Drucktaste ausgebildet, welche von dem Drehstück, z. B. in Form eines die Drucktaste aufnehmenden ringförmigen Handrades, umgeben ist, so daß die Drucktaste innerhalb dieses Drehstücks versenkbar angeordnet werden kann. Dadurch ist ein unbeabsichtigtes Verstellen der Schließkraft während der Betätigung der Drucktaste vermieden und gleichzeitig eine deutliche optische und taktile Feststellung des Schaltzustandes möglich. In der Lüftstellung mit gelöster Rastvorrichtung überwiegt die Kraft des Schaltfederelementes die Schließkraft des Schließfederelementes, wodurch der Ventilteller von seinem Sitz angelüftet wird. In der Schließstellung mit eingerasteter Rastvorrichtung ist durch das Hineindrücken der Drucktaste der Ventilschaft auf den Sitz zu verschoben, so daß der Ventilteller unabhängig und nur unter der eingestellten Kraft des Schließfederelementes dem Sitz anliegt.

In alternativer Ausbildung der Erfindung weist die Schaltvorrichtung einen das äußere Ende des Ventilschaftes durchquerenden, in Langlöchern des Drehstückes geführten Schaltstift auf, und das Betätigungsstück ist als ein in Schlitzen des Drehstückes geführter Schaltbügel ausgebildet, der an seinen Schenkeln den Schaltstift aufnimmt und mit nockenförmigen Stützkanten gegenüber dem Drehstück versehen ist. Bei dieser Ausbildung nimmt der Ventilschaft unter der Kraft der Schaltfeder die Schließstellung ein und wird bei Umlegen des Schaltbügels gegen die Kraft der Schaltfeder in die Lüftstellung gebracht. In seinen Endlagen bietet der Schaltbügel verschiedene Seiten dem Blick dar, die mit Farb- oder Schriftkennzeichnung zur sinnfälligen Anzeige des Schaltzustandes versehen sein können.

Ausführungsbeispiele der Erfindung sind in der Zeichnung schematisch dargestellt und im folgenden näher erläutert. Es zeigen
- Fig. 1: Ein Umschaltventil mit Drucktastenschaltung,
- Fig. 2: ein Umschaltventil mit Hebelschaltung,
- Fig. 3: die Seitenansicht der Schaltbügel-Gruppe nach Fig. 2.

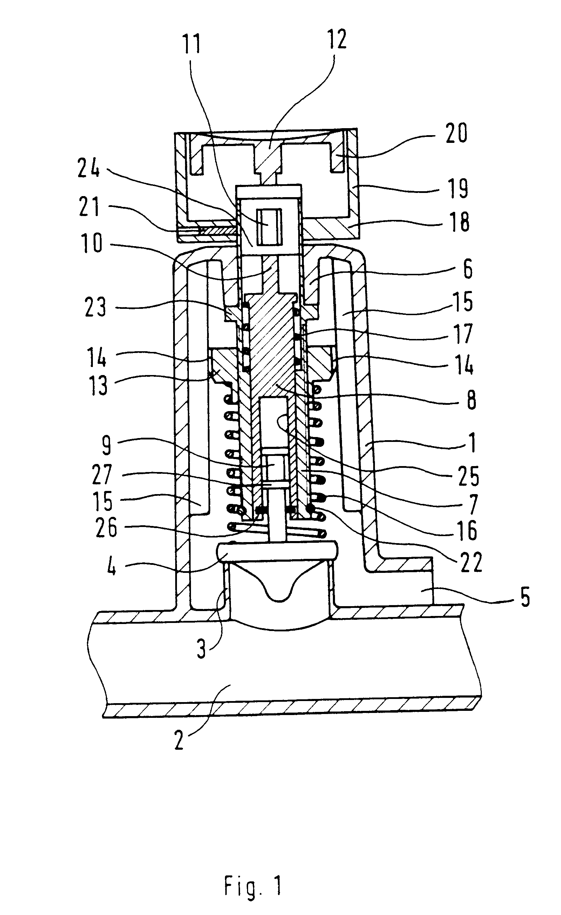

In Figur 1 ist ein Überdruckventil dargestellt, dessen Gehäuse (1) auf einem Teilstück einer Atemgasleitung (2) aufgesetzt ist. Ein kraterförmiger Sitz (3) für einen Ventilteller (4) bildet in der nicht dargestellten angelüfteten Position die Strömungsverbindung zwischen Atemgasleitung (2) und einer in die Umgebung mündenden Abströmöffnung (5). In das Ventilgehäuse (1) ragt von seiner oberen, mit einem in das Innere des Gehäuses (1) gerichteten Kragen (6) versehenen Öffnung eine Ventilspindel (7) hinein, die über einen Ventilschaft (8) gezogen ist. In dessen unteren Ende ist der an einem Stempel (9) in einer Schleppverbindung aufgenommene Ventilteller (4) eingelassen. Am oberen Ende bildet ein Steg (10), der eine Schaltvorrichtung in Form von Rastnocken (11) trägt, die Fortsetzung des Ventilschaftes (8) zu einem als Drucktaste (12) ausgebildeten Betätigungsstück. Die mit einem Außengewinde versehene Ventilspindel (7) ist von einem mit einem Innengewinde versehenen Haltekragen (13) umgeben, welcher an seiner äußeren Peripherie Führungsfahnen (14) besitzt, die in längs der Ventilspindel (7) ausgerichteten Führungsnuten (15) des Gehäuses (1) aufgenommen sind.

Zwischen dem Ventilteller (4) und dem Haltekragen (13) ist eine Schließfeder (16) angeordnet. Zwischen der Ventilspindel (7) und dem Ventilschaft (8) ist in einer entsprechenden Ausnehmung eine Schaltfeder (17) eingespannt. Ihre Kraftrichtung geht auf eine Verschiebung des Ventilschaftes (8) relativ zur Ventilspindel (7) in Öffnungsrichtung des Ventiltellers (4).

Der aus dem Gehäuse (1) über den Kragen (6) in den Außenraum ragende Teil der Ventilspindel (7) ist außen mit einem knopfförmigen Drehstück (18) versehen, dessen Griffrand (19) den Randbereich (20) der Drucktaste (12) umschließt. Die Befestigung des Drehstücks (18) an der Ventilspindel (7) geschieht mit der Schraube (21). Innen enthält die Ventilspindel (7) ein Rastgehäuse (24) als Gegenstück zu den Rastnocken (11) des Ventilschaftes (8).

Das in Schließstellung dargestellte Überdruckventil hält die Atemgasleitung (2) an dem Sitz (3) so lange verschlossen, wie ein durch die Federkraft der Schließfeder (16) bestimmter Öffnungsdruck nicht überschritten wird. Bei Überschreiten dieses Öffnungsdruckes wird der Ventilteller (4) entgegen der Federkraft der Schließfeder (16) angehoben und gibt den Strömungsweg aus der Atemgasleitung (2) über den freien Sitz (3) in die Abströmöffnung (5) frei. Durch Verdrehen des Drehstücks (18) in Uhrzeigerrichtung wird der Haltekragen (13) entlang der Ventilspindel (7) in Richtung auf den Ventilteller (4) geschraubt, wobei er in den Führungsnuten (15) in Längsrichtung gleitet. Die Schließkraft des Ventiltellers (4) wird dadurch erhöht. Bei Drehung des Drehstücks (18) entgegen dem Uhrzeigersinn wird die Schließkraft entsprechend verringert. Am ventiltellerseitigen Ende der Ventilspindel (7) dient ein Sicherungsring (22), in der anderen Richtung ein ringförmiger Absatz (23) auf der Ventilspindel (7) als Hubbegrenzung für den Weg des Haltekragens (13).

Bei Betätigung der Drucktaste (12) lösen sich die innerhalb des Rastgehäuses (24) beweglichen Rastnocken (11) und geben damit den Ventilschaft (8) frei, so daß dieser unter Krafteinwirkung der Schaltfeder (17) entgegen der Federkraft der Schließfeder (16) in Öffnungsrichtung des Ventiltellers (4) gehoben wird. Der Hubweg ist so bemessen, daß der Ventilschaft (8) mittels seines in der Stempelausnehmung (25) befindlichen Mitnehmerringes (26) den Stempel (9) an dessen Führungsring (27) mitnimmt und so den Ventilteller (4) von seinem Sitz (3) abhebt. Bei erneuter Betätigung der Drucktaste (12) wird der Ventilschaft (8) gegen die Kraft der Schaltfeder (17) wieder in die dargestellte Schließstellung verschoben, wobei die Rastnocken (11) in ihre Raststellung im Rastgehäuse (24) einrasten und die Teile in der Schließstellung verriegeln.

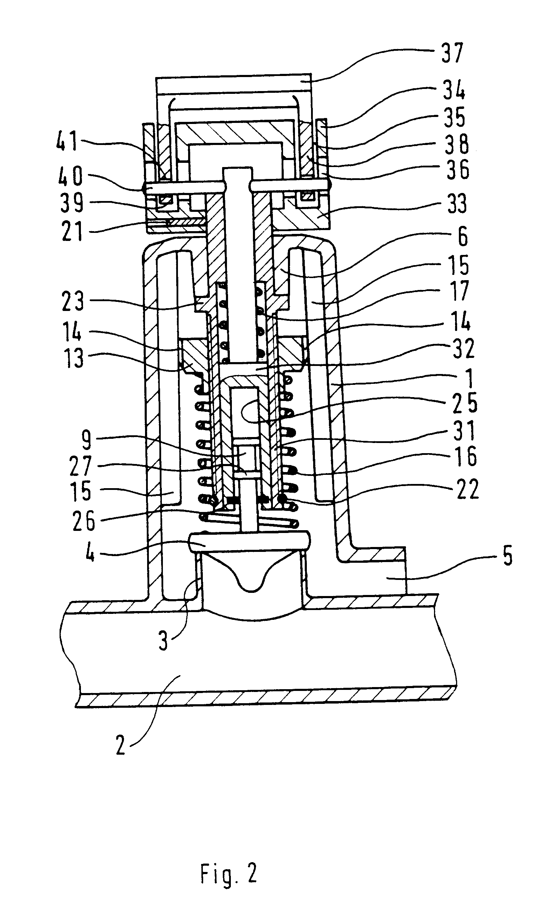

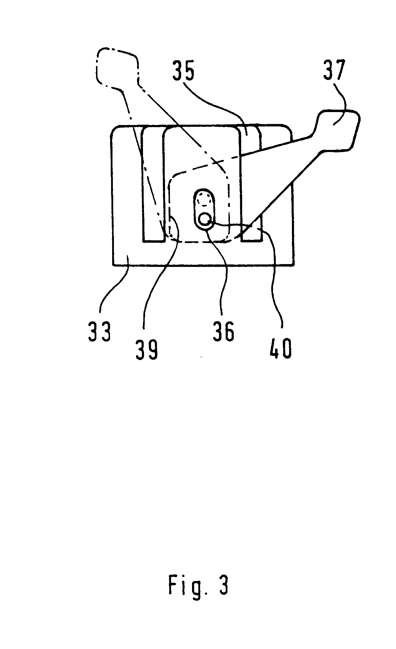

Soweit das Überdruckventil nach Figur 2 und 3 in seinen Teilen mit der bereits beschriebenen Ausführung nach Figur 1 übereinstimmt, sind dieselben Bezugszeichen verwendet. Die Schaltfeder (17) zwischen Ventilspindel (31) und Ventilschaft (32) stützt sich an diesen Teilen in der Weise ab, daß die Kraftrichtung auf eine Verschiebung des Ventilschaftes (32) in Schließrichtung des Ventiltellers (4) geht. Das Drehstück (33) enthält in seinem Griffrand (34) zwei parallele Schlitze (35) und senkrecht dazu zwei Langlöcher (36). In den Schlitzen (35) ist als Betätigungsstück und Schaltvorrichtung ein Schaltbügel (37) mit seinen Schenkeln (38) beweglich geführt und dabei mit nockenförmig ausgebildeten Stützkanten (39) am Ende der Schenkel (38) auf dem Grund der Schlitze (35) abgestützt. Ein Schaltstift (40) durchsetzt die Langlöcher (36), die Schenkel (38) und das obere Ende des Ventilschaftes (32). Die Durchdringungsöffnung (41) in jedem Schenkel (38) ist dabei so angeordnet, daß sie von den Abschnitten der Stützkante (39) verschiedene Abstände hat. In der dargestellten Schließstellung ist der Abstand des Schaltstiftes (40) zu den Stützkanten (39) gering und der Ventilschaft (32) unter der Kraft der Schaltfeder (17) dem Sitz (3) genähert. Mit dem Umlegen des Schaltbügels (37) in seine andere in Fig. 3 strichpunktiert angedeutete Endlage, in der der Abstand des Schaltstiftes (40) zu den Stützkanten (39) größer ist, wird, unter Verschiebung des Schaltstiftes (40) in den Langlöchern (36), der Ventilschaft (32) gegen die Kraft der Schaltfeder (17) von dem Sitz (3) entfernt und damit die Lüftstellung eingeschaltet.

## Patentansprüche

1. Überdruckventil für strömende Medien mit einem in seiner Schließkraft einstellbaren Verschlußelement (4), welches durch Betätigen einer Verstelleinrichtung von seinem Sitz (3) anlüftbar ist, welche eine bistabile Schaltvorrichtung (11, 24; 37) enthält, durch die das Verschlußelement (4) in einer Lüftstellung angelüftet und in einer Schließstellung unter Einwirkung der voreingestellten Schließkraft auf den Sitz (3) gedrückt ist,
dadurch gekennzeichnet, daß die Verstelleinrichtung innerhalb einer drehbaren Ventilspindel (7; 31) und mit dieser über die Schaltvorrichtung verbunden einen mittels eines Betätigungsstückes (12; 37) hubbeweglichen Ventilschaft (8; 32) enthält, an dem über eine Schleppverbindung (26, 27) ein Ventilteller (4) als Verschlußelement angeordnet ist, wobei zur Erzeugung der Schließkraft ein Schließfederelement (16) zwischen dem Ventilteller (4) und einem längs der Ventilspindel (7; 31) einstellbaren Haltekragen (13), und für die Hubbewegung zwischen der Ventilspindel (7; 31) und dem Ventilschaft (8; 32) ein in der Achse der Hubbewegung wirkendes Schaltfederelement (17) angreifen.

2. Überdruckventil nach Anspruch 1, dadurch gekennzeichnet, daß auf der mit einem Drehstück (18; 33) als Handhabe ausgestatteten und mit einem Außengewinde versehenen Ventilspindel (7; 31) der mit einem entsprechenden Innengewinde versehene Haltekragen (13) angeordnet ist, der wiederum längs der Ventilspindel (7; 31) verdrehsicher in Führungsnuten (15) geführt ist.

3. Überdruckventil nach einem der Ansprüche 1 bis 2, dadurch gekennzeichnet, daß die Schaltvorrichtung ein von der Ventilspindel (7) getragenes Rastgehäuse (24) und in dieses eingreifende, mit dem Ventilschaft (8) verbundene Rastnocken (11) aufweist, und daß das Betätigungsstück als Drucktaste (12) zur Betätigung der Schaltvorrichtung (11, 24) ausgebildet ist, welche von dem Drehstück (18) umgeben ist.

4. Überdruckventil nach einem der Ansprüche 1 bis 2, dadurch gekennzeichnet, daß die Schaltvorrichtung einen das äußere Ende des Ventilschaftes (32) durchquerenden, in Langlöchern (36) des Drehstückes (33) geführten Schaltstift (40) aufweist, und daß das Betätigungsstück als ein in Schlitzen (35) des Drehstückes (33) geführter Schaltbügel (37) ausgebildet ist, der an seinen Schenkeln (38) den Schaltstift (40) aufnimmt und mit nockenförmigen Stützkanten (39) gegenüber dem Drehstück (33) versehen ist.

## Claims

1. Excess-pressure valve for flowing media with a closure element (4) adjustable in its closing force, which, through actuation of an adjusting device, can be lifted up from its seat (3), which contains a two-state switching device (11, 24; 37), by means of which the closure element (4) is lifted up in an air position and is pressed on to the seat (3) in a closing position under the action of the preset closing force, characterized in that the adjusting device within a rotatable valve spindle (7; 31) and connected thereto by way of the switching device contains a valve shaft (8; 32) lift-moveable by means of an actuation piece (12; 37), on which shaft a valve plate (4) as closure element is arranged by way of a towing connection (26, 27), whereby to generate the closing force a closing spring element (16) engages between the valve plate (4) and a holding collar (13) adjustable along the valve spindle (7; 31), and for the lifting movement between the valve spindle (7; 31) and the valve shaft (8; 32) a switching spring element (17), acting in the axis of the lifting movement, engages.

2. Excess-pressure valve according to claim 1, characterized in that the holding collar (13) provided with an appropriate inner thread is arranged on the valve spindle (7; 31) equipped with a rotary piece (18; 33) as handle and provided with an outer thread, the holding collar in turn being guided along the valve spindle (7; 31) in a rotation-resistant manner in guide grooves (15).

3. Excess-pressure valve according to one of claims 1 to 2, characterized in that the switching device has a catch housing (24) carried by the valve spindle (7) and a catch cam (11) engaging into the housing and connected to the valve shaft (8), and in that the actuation piece is constructed as a push-button (12) for the actuation of the switching device (11, 24) which is surrounded by the rotary piece (18).

4. Excess-pressure valve according to one of claims 1 to 2, characterized in that the switching device has an operating pin (40) traversing the outer end of the valve shaft (32) and guided in longitudinal holes (36) of the rotary piece (33), and in that the actuation piece is constructed as a switch clamp (37) guided into slots (35) of the rotary piece (33), which clamp takes up the operating pin (40) at its limbs (38) and is provided with cam-shaped supporting edges (39) opposite the rotary piece (33).

## Revendications

1. Soupape de sûreté pour fluides s'écoulant, pourvue d'un élément de fermeture (4) dont la force de fermeture peut être réglée, qui peut être placé en position de mise à l'air libre, au niveau de son siège (3), par actionnement d'un dispositif de réglage contenant un dispositif de commutation (11, 24 ; 37) bistable, grâce auquel l'élément de fermeture (4) est soulevé dans une position de mise à l'air libre et est appuyé, en position de fermeture, sur le siège (3), sous l'action d'une force de fermeture préréglée, caractérisée en ce que le dispositif de réglage, à l'intérieur d'une tige de soupape rotative (7 ; 31), et relié à cette dernière par l'intermédiaire d'un dispositif de commutation, contient une queue de soupape (8 ; 32) pouvant effectuer une course d'élévation grâce à un organe d'actionnement (12 ; 37), et à laquelle est associée, par une liaison par traction (26, 27), une tête de soupape (4) servant d'élément de fermeture, un élément de fermeture à ressort (16) étant prévu, pour produire la force de fermeture, entre la tête de soupape (4) et un collet de maintien (13) réglable le long de la tige de soupape (7 ; 31) et un élément de commutation à ressort (17), agissant dans l'axe de la course d'élévation, étant disposé entre la tige de soupape (7 ; 31) et la queue de soupape (8 ; 32).

2. Soupape de sûreté selon la revendication 1, caractérisée en ce que le collet de maintien (13), pourvu d'un filet intérieur adapté, est monté sur la tige de soupape (7 ; 31), pourvue d'un organe rotatif (18 ; 33) servant de manette et d'un filet extérieur, et est guidé, à son tour, dans des rainures de guidage (15), le long de la tige de soupape (7 ; 31) et sans possibilité de torsion.

3. Soupape de sûreté selon l'une des revendications 1 et 2, caractérisée en ce que le dispositif de commutation présente un boîtier d'encliquetage (24) porté par la tige de soupape (7) et des ergots d'encliquetage (11) liés à la queue de soupape (8) et s'engageant dans celui-ci, et en ce que l'organe d'actionnement est conformé en bouton-poussoir (12) pour l'actionnement du dispositif de commutation (11, 24), bouton qui est entouré par l'organe rotatif (18).

4. Soupape de sûreté selon l'une des revendications 1 et 2, caractérisée en ce que le dispositif de commutation présente un doigt de commutation (40) traversant l'extrémité externe de la queue de soupape (32) et guidé dans des perçages oblongs (36) de l'organe rotatif (33) et en ce que l'organe d'actionnement est conformé en étrier de commutation (37), guidé dans des fentes (35) de l'organe rotatif (33), qui reçoit le doigt de commutation (40) au niveau de ses branches (38) et qui est pourvu d'arêtes d'appui (39), vis-à-vis de l'organe rotatif (33), en forme d'ergots.
